# EUROPEAN PATENT APPLICATION

(11) **EP 2 589 382 A1**
(43) Date of publication of application: **08.05.2013**
(21) Application number: 11800122.1
(22) Date of filing: 10.06.2011
(51) Int. Cl.: A61K 31/205, A61K 31/185, A61P 13/12, A61P 9/10, A61P 9/00, A61P 3/10

(54) **PHARMACEUTICAL COMPOSITION COMPRISING LEVOCARNITINE AND DOBESILATE**

(30) Priority: 29.06.2010 CN 201010215998
(71) Applicant: Liaoning Sebest Pharmaceutical Co., Ltd., Shenyang, Liaoning 110002 (CN); Nankai Share Pharmaceutical Co., Ltd., Inner Mongolia 028100 (CN); Xizang Linzhi Parkson Pharmaceutical Co., Ltd., Tibet 860000 (CN); Tianjin Nankai Share Pharmaceutical Science & Technology Co., Ltd., Tianjin 300384 (CN)
(72) Inventor: WANG, Yong, Shanghai 200030 (CN); ZHAO, Xin, Shenyang Liaoning 110023 (CN); CHEN, Mo, Shenyang City Liaoning 110003 (CN); TIAN, Ning, Shenyang Liaoning 110022 (CN); WANG, Shujun, Shenyang Liaoning 110015 (CN)
(74) Representative: Hoffmann, Matthias
(86) International application number: PCT/CN2011/075570
(87) International publication number: WO 2012/000377

(57) **Abstract**

A pharmaceutical composition comprising levocarnitine and dobesilate is provided. Also provided are a method for regulating the level of serum creatinine and/or blood urea nitrogen and a method for treating and/or preventing the diseases influencing renal function, for example, including but not limiting to various primary, secondary nephritis, renal lesions, renal insufficiency, nephritic syndrome, even renal failure, uremia, as well as the complications induced by the diseases and/or disorders influencing renal function for example, such as cardiovascular diseases, diabetes and the like.

## Description

### FIELD OF THE INVENTION

The present invention relates to a pharmaceutical composition comprising levocarnitine and dobesilate. The invention also relates to a method for regulating the level of serum creatinine (Scr) and/or blood urea nitrogen (BUN) using the pharmaceutical composition. The invention further relates to a method for treating and/or preventing the diseases and/or disorders influencing renal function, for example, including but not limited to various primary and secondary nephritis, renal lesions, renal insufficiency, nephritic syndrome, and even renal failure and uremia, as well as the complications induced by the diseases and/or disorders influencing renal function, such as cardiovascular diseases, diabetes and the like.

### BACKGROUND OF THE INVENTION

The chemical name of levocarnitine is (*R*)-3-carboxy-2-hydroxy-N,N,N-trimethyl-1-propanaminium hydroxide, and its structure is shown by the following formula.

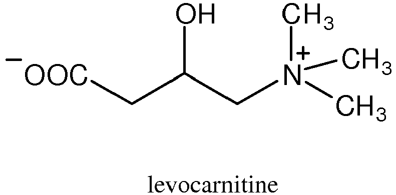

It is a class of special amino acid widely present in an organism as an essential cofactor for the metabolism of fatty acids. It can promote fatty acids into the tricarboxylic acid cycle via β-oxidation to generate energy, and thus play a role in the improvement of myocardial ischemia and the protection of liver. At present, clinically levocarnitine is primarily used as an adjuvant treatment for diseases such as complications related to maintenance hemodialysis, cardiovascular diseases, diabetes, and the like.

Recent research has shown that levocarnitine is correlated with metabolic diseases, cardiovascular diseases, nervous system diseases, liver diseases and renal diseases (Yanhong QIAO et al., Levocarnitine and chronic renal failure, Journal of Changzhi Medical College, April 2008, Vol 22, Issue 2, pp. 151-153; Yongxu SUN et al, Relation between levocarnitine and renal disease, Progress in Modern Biomedicine, 2007, Vol. 7, No. 11, pp1750-1753). Renal diseases and the clinical therapy of the renal diseases have an important influence on the level of levocarnitine in a human body as levocarnitine is metabolized mainly in kidney. The deficiency of levocamitine is associated directly with many diseases. When levocarnitine is deficient in a human body, clinical symptoms such as hypotension, anemia, myasthenia and fatigue and the like may often occur during dialysis. The supplement of levocarnitine can not only improve the anemia of a chronic renal failure patient, also significantly reduce the level of serum cholesterol, triglyceride, low density lipoprotein, increase the level of high density lipoprotein, improve lipid metabolism, myocardial function, nutritional status, and relieve skeletal muscle symptoms and the like, thus has a remarkable clinical importance.

Animal studies have shown that levocarnitine can reduce serum creatinine (Scr) and blood urea nitrogen (BUN) (Dexuan WANG, Protection of Levocarnitine on Renal Ischemic /Reperfusion Injury in Rats, JOURNAL OF CHINESE MICROCIRCULATION, April, 2009, Vol 13, Issue 2, pp 105-107). It follows that levocarnitine may be, to some extent, protective to the renal function in an ischemic / reperfused rat. In addition, levocarnitine can reduce the content of malonaldehyde (MDA) in renal tissue, increase the content of reduced glutathione(GSH), thus its mechanism is related with the protection from oxygen free radical by an enhanced activity of superoxide dismutase (SOD) and a decreased peroxidation degree of kidney.

The chemical name of dobesilate is 2,5-dihydroxybenzenesulfonate, and its structure is as follows: 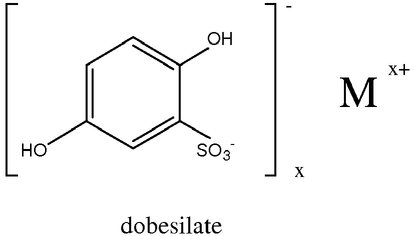 wherein M is a cation of x valent.

Dobesilate is a medicament for improving microcirculation. It can reduce the permeability and fragility of capillary, suppress platelet aggregation, decrease blood viscosity, enhance the flexibility of hemoglobin, and improve lymph return. At present, clinically dobesilate is mainly useful for the treatment of microangiopathy, retinopathy, glomerulosclerosis, varices syndrome, and microcirculation disorders associated with chronic organic diseases (such as hypertension, arteriosclerosis and hepatocirrhosis), and the like.

More and more comprehensive studies in recent years have found that dobesilate not only can alleviate angioendothelial damage and apoptosis, suppress the synthesis of basement membrane collagens, and prevent capillary basement membrane from thickening, but also can decrease the level of cholesterol, the viscosity of whole blood and plasma, and can reduce the deposition of extracellular matrix, directly alleviate renal damage, and improve renal function by suppressing the expression of type-IV collagens (Xinghua CHEN etc., The progress in the clinical application of calcium dobesilate, THE JOURNAL OF PRACTICAL MEDICINE, 2007, Vol. 23, Issue 4, pages 593-595). Therefore, recently it has also been used clinically in the treatment of renal diseases, chronic venous insufficiency, thrombotic diseases and some cardiac diseases etc.

Animal studies have shown that dobesilate such as calcium dobesilate can improve renal function, reduce notably the content of thromboxane Az in the homogenate of renal cortex, alleviate distinctly the pathological changes of residual renal tissue and thereby retard the progress of glomerular fibrosis and chronic renal failure by decreasing the level of Scr and BUN (Dan WANG, "The influence of calcium dobesilate on the vaso-active substances of rats in chronic renal failure model", CHINA PHARMACY, 2005, Vol. 16, Issue 11, pages 821-823).

Scr and BUN are the end products of the *in vivo* metabolism of nitrogen-containing organic substances, and the levels of Scr and BUN are major indications of renal function. In the case of normal renal function, these small molecules are filtered off from glomerular; while in the case of abnormal renal function, as glomerular filtration rate decreases, the excretion of Scr and BUN reduces. When the glomerular filtration rate decreases to the range from 1/2 to 1/3 of the normal value, the concentrations of Scr and BUN in blood gradually increase, finally leading to the development of renal diseases.

A number of diseases and/or disorders influencing renal function may increase the levels of Scr and/or BUN, for example, including but not limited to various primary and secondary nephritis, renal lesions, renal insufficiency, nephritic syndrome, and even renal failure and uremia, as well as the complications induced by the diseases influencing renal function, such as cardiovascular diseases, diabetes and the like. If a medicament can reduce the levels of Scr and/or BUN, then to a certain degree it can improve renal function and alleviate the severity of the diseases, thereby to treat and/or prevent the diseases and/or disorders to some extent.

Chronic renal failure (CRF) is a common disease among renal diseases. In China, statistically, there are from 90 to 100 persons in every 1000,000 persons per year entering renal failure phase.

Therefore, an object of the invention is to provide a pharmaceutical composition capable of effectively regulating the levels of Scr and/or BUN so as to be useful for the treatment and/or prevention of the above-mentioned diseases and/or disorders influencing renal function.

### Summary of the invention

In one aspect, the invention relates to a pharmaceutical composition comprising levocarnitine and dobesilate.

In another aspect, the invention particularly relates to a pharmaceutical composition consisting of levocarnitine and dobesilate together with pharmaceutically acceptable excipient.

In another aspect, the present invention relates to a method for regulating the levels of serum creatinine (Scr) and/or blood urea nitrogen (BUN), wherein the method comprises administering the pharmaceutical composition according to the invention to a patient in need thereof.

In another aspect, the invention relates to a method for treating and/or preventing the diseases influencing renal function, for example, including but not limited to various primary and secondary nephritis, renal lesions, renal insufficiency, nephritic syndrome, and even renal failure and uremia, as well as the complications induced by the diseases and/or disorders influencing renal function, such as cardiovascular diseases, diabetes and the like, wherein the method comprises administering the pharmaceutical composition according to the invention to a patient in need thereof.

In another aspect, the invention particularly relates to a method for treating and/or preventing chronic renal failure, wherein the method comprises administering the pharmaceutical composition according to the invention to a patient in need thereof.

In another aspect, the present invention relates to use of levocamitine and dobesilate in the preparation of a medicament for treating and/or preventing the diseases influencing renal function, for example, including but not limited to various primary and secondary nephritis, renal lesions, renal insufficiency, nephritic syndrome, and even renal failure and uremia, as well as the complications induced by the diseases and/or disorders influencing renal function, such as cardiovascular diseases, diabetes and the like.

In another aspect, the invention particularly relates to use of levocarnitine and dobesilate in the preparation of a medicament for treating and/or preventing chronic renal failure.

In another aspect, the present invention relates to a pharmaceutical preparation comprising levocarnitine and dobesilate.

In another aspect, the invention particularly relates to a pharmaceutical preparation consisting of levocarnitine and dobesilate together with pharmaceutically acceptable excipient.

### Detailed description of the invention

Surprisingly, the inventors have found that the use of levocarnitine and dobesilate in combination has a synergistic effect.

Therefore, in one aspect, the invention relates to a pharmaceutical composition comprising levocarnitine and dobesilate.

In another aspect, the invention particularly relates to a pharmaceutical composition consisting of levocarnitine and dobesilate together with pharmaceutically acceptable excipient.

In another aspect, the pharmaceutical composition according to the invention is useful for regulating the levels of serum creatinine and/or blood urea nitrogen.

Therefore, in another aspect, the present invention further relates to a method for regulating the levels of serum creatinine (Scr) and/or blood urea nitrogen (BUN), wherein the method comprises administering the pharmaceutical composition according to the invention to a patient in need thereof.

In another aspect, the invention further relates to a method for treating and/or preventing the diseases influencing renal function, for example, including but not limited to various primary and secondary nephritis, renal lesions, renal insufficiency, nephritic syndrome, and even renal failure and uremia, as well as the complications induced by the diseases and/or disorders influencing renal function, such as cardiovascular diseases, diabetes and the like, wherein the method comprises administering the pharmaceutical composition according to the invention to a patient in need thereof.

In another aspect, the invention particularly relates to a method for treating and/or preventing renal failure, wherein the method comprises administering the pharmaceutical composition according to the invention to a patient in need thereof.

In another aspect, the invention particularly relates to a method for treating and/or preventing chronic renal failure, wherein the method comprises administering the pharmaceutical composition according to the invention to a patient in need thereof.

In another aspect, the invention particularly relates to a method for treating and/or preventing primary nephritis, wherein the method comprises administering the pharmaceutical composition according to the invention to a patient in need thereof.

In another aspect, the invention particularly relates to a method for treating and/or preventing secondary nephritis, wherein the method comprises administering the pharmaceutical composition according to the invention to a patient in need thereof.

In another aspect, the invention particularly relates to a method for treating and/or preventing renal lesions, wherein the method comprises administering the pharmaceutical composition according to the invention to a patient in need thereof.

In another aspect, the invention particularly relates to a method for treating and/or preventing nephritic syndrome, wherein the method comprises administering the pharmaceutical composition according to the invention to a patient in need thereof.

In another aspect, the invention particularly relates to a method for treating and/or preventing renal failure, wherein the method comprises administering the pharmaceutical composition according to the invention to a patient in need thereof.

In another aspect, the invention particularly relates to a method for treating and/or preventing uremia, wherein the method comprises administering the pharmaceutical composition according to the invention to a patient in need thereof.

In another aspect, the invention particularly relates to a method for treating and/or preventing complications induced by the diseases influencing renal function (such as cardiovascular diseases, diabetes and the like), wherein the method comprises administering the pharmaceutical composition according to the invention to a patient in need thereof.

In another aspect, the present invention relates to use of levocarnitine and dobesilate in the preparation of a medicament for treating and/or preventing the diseases influencing renal function, for example, including but not limited to various primary and secondary nephritis, renal lesions, renal insufficiency, nephritic syndrome, and even renal failure and uremia, as well as the complications induced by the diseases and/or disorders influencing renal function, such as cardiovascular diseases, diabetes and the like.

In another aspect, the invention particularly relates to use of levocarnitine and dobesilate in the preparation of the medicament for treating and/or preventing chronic renal failure.

In another aspect, the present invention relates to a pharmaceutical preparation comprising levocarnitine and dobesilate.

In another aspect, the invention particularly relates to a pharmaceutical preparation consisting of levocarnitine and dobesilate together with pharmaceutically acceptable excipient.

As used herein, levocarnitine has a chemical structure of (R)-3-carboxy-2-hydroxy-N,N,N-trimethyl-1-propanaminium hydroxide. For the purpose of the invention, the term levocarnitine includes levocarnitine and pharmaceutically acceptable salts thereof. In one particular embodiment, the levocarnitine is a pharmaceutically acceptable salt formed by levocarnitine and a pharmaceutically acceptable acid or base. In one particular embodiment, the levocarnitine presents in the form of an inner salt.

As used herein, dobesilate includes various pharmaceutically acceptable salts of dobesilate. In one particular embodiment, the dobesilate is a salt formed by dobesilate and alkali or alkaline earth metal cations. In one particular embodiment, the dobesilate is calcium dobesilate.

As used herein, the term "pharmaceutically acceptable salts" includes acid addition salts and base addition salts. Such acid addition salts include the salts formed with a pharmaceutically acceptable organic or inorganic acid, such as hydrochloric acid, hydrobromic acid, hydriodic acid, hydrofluoric acid, sulfuric acid, citric acid, maleic acid, acetic acid, lactic acid, nicotinic acid, succinic acid, oxalic acid, phosphoric acid, malonic acid, salicylic acid, phenylacetic acid, stearic acid, methanesulfonic acid, picric acid, tartaric acid and the like. Such base addition salts include the salts formed with a pharmaceutically acceptable organic or inorganic base, such as pyridine, ammonium, piperazine, diethylamine, niacinamide, formic acid, alkali metal, alkaline earth metal, cinnamic acid, methylamine, triethylamine, dimethylamine, and tris(methylol)aminomethane salts and the like. The salts formed with an inorganic base are preferably alkali or alkaline earth metal salts, such as sodium salts, potassium salts, calcium salts, magnesium salts, zinc salts and lithium salts. Other pharmaceutically acceptable salts are known by those skilled in the art.

The term "pharmaceutically acceptable excipient" means a pharmaceutically acceptable material, composition or carrier, for example, liquid or solid fillers, dilutents, excipients, solvents or encapsulating materials. Each of the components must be "pharmaceutically acceptable", which means it is compatible with the other components of the pharmaceutical preparation. It also must be suitable for contacting with the organs or tissues of a human or animal without any undue toxicity, stimulus, anaphylaxis, immunogenicity or any other problems or complications, and commensurate with a reasonable benefit/risk ratio. *(See,* Remington: The Science and Practice of Pharmacy, 21st Ed.; Lippincott Williams & Wilkins: Philadelphia, PA, 2005; Handbook of Pharmaceutical Excipients, 5th Ed.; Rowe et al. eds, The Pharmaceutical Press and the American Pharmaceutical Association: 2005; and Handbook of Pharmaceutical Additives, 3rd Ed.; Ash and Ash ed., Gower Publishing Company: 2007; Pharmaceutical Preformulation and Formulation, Gibson ed., CRC Press LLC: Boca Raton, FL, 2004).

As used herein, the term "synergistic effect" or "synergistically" means that the effect of a combination is superior to the sum of the effects of each individual components alone. In particular, it means that the combination of levocarnitine and dobesilate according to the invention has better effects than the effects resulted from using these two components alone for the prevention and/or treatment of the diseases and/or disorders according to the invention.

As known by those skilled in the art, in the pharmaceutical composition according to the invention, levocarnitine and dobesilate may be formulated for the same or different administration route. Similarly, levocarnitine and dobesilate may be formulated in the same or different dosage forms. As known by those skilled in the art, the levocarnitine and dobesilate according to the invention may be administrated simultaneously, successively, or intermittently, as long as the interval therebetween does not allow the combined administration lose its effects.

In one particular embodiment, in the pharmaceutical composition of the present invention, levocarnitine and dobesilate is administrated by the same administration route, preferably by oral administration. In one particular embodiment, in the pharmaceutical composition of the present invention, levocarnitine and dobesilate is formulated into the same dosage form, such as injections, granules, capsules, tablets and oral solutions, preferably tablets. In one particular embodiment, in the pharmaceutical composition of the present invention, levocarnitine and dobesilate is administrated simultaneously.

In one particular embodiment, the invention provides a pharmaceutical preparation comprising levocarnitine and dobesilate. In one particular embodiment, the invention provides a pharmaceutical preparation consisting of levocarnitine and dobesilate together with pharmaceutically acceptable excipients. In one particular embodiment, the pharmaceutical preparation is in the form of capsules. In one particular embodiment, the pharmaceutical preparation is in the form of tablets. In one particular embodiment, the pharmaceutical preparation is in the form of oral solutions. In one particular embodiment, the pharmaceutical preparation is in the form of injections.

In one particular embodiment, in the pharmaceutical composition or pharmaceutical preparation of the present invention, the mass ratio of levocarnitine to dobesilate is from about 1:10 to about 10:1. In one particular embodiment, in the pharmaceutical composition or pharmaceutical preparation of the present invention, the mass ratio of levocarnitine to dobesilate is about 1:10, 1:6, 1:3, 1:2, 1:1, 2:1, 3:1, 6:1 or 10:1. In one particular embodiment, in the pharmaceutical composition or pharmaceutical preparation of the present invention, the mass ratio of levocarnitine to dobesilate is from about 1:3 to about 3:1. In one particular embodiment, in the pharmaceutical composition or pharmaceutical preparation of the present invention, the mass ratio of levocarnitine to dobesilate is from about 1:2 to about 2:1. In one particular embodiment, in the pharmaceutical composition or pharmaceutical preparation of the present invention, the mass ratio of levocarnitine to dobesilate is about 1:1.25, about 1:1.5, about 1:1.75, about 1.25:1, about 1.5:1, or about 1.75:1. In one particular embodiment, in the pharmaceutical composition or pharmaceutical preparation of the present invention, the mass ratio of levocarnitine to dobesilate is about 1:1.

Accordingly, in one particular embodiment of the methods or uses according to the present invention, the ratio of the amount of levocarnitine to dobesilate (mass ratio) is from about 1:10 to about 10:1. In one particular embodiment of the methods or uses of the present invention, the ratio of the amount of levocarnitine to dobesilate (mass ratio) is about 1:10, 1:6, 1:3, 1:2, 1:1, 2:1, 3:1, 6:1 or 10:1. In one particular embodiment of the methods or uses according to the present invention, the ratio of the amount of levocarnitine to dobesilate (mass ratio) is from about 1:3 to about 3:1. In one particular embodiment of the methods or uses according to the present invention, the ratio of the amount of levocarnitine to dobesilate (mass ratio) is from about 1:2 to about 2:1. In one particular embodiment of the methods or uses according to the present invention, the ratio of the amount of levocarnitine to dobesilate (mass ratio) is about 1:1.25, about 1:1.5, about 1:1.75, about 1.25:1, about 1.5:1, or about 1.75:1. In one particular embodiment of the methods or uses according to the present invention, the ratio of the amount of levocarnitine to dobesilate (mass ratio) is about 1:1.

Those skilled in the art will understand that based on the present disclosure a person skilled in the art would be able to adjust the dosages according to the known factors such as the age, health status and body weight of the patient to be treated, the progress of diseases, other types of concomitant therapies, the frequency of treatments, and the desired effects. For example, in the composition or preparation according to the present invention, the dosage of levocarnitine may be from 0.25 g/ day to 4g / day, preferably from 0.5 g/ day to 1 g/ day; and the dosage of dobesilate is from 0.25 g/ day to 3 g/ day, preferably from 0.75 g/ day to 1 g / day for treating and/or preventing the diseases and/or disorders described herein.

Hereafter, the present invention is more specifically explained by the following examples. However, it should be understood that the scope of the present invention is not limited thereto.

### Examples

In the following examples, experimental animals and reagents were obtained from:
Levocarnitine: Northeast General Pharmaceutical Factory (inner salts)
calcium dobesilate: BEIJING JIAN LI PHARMACEUTICAL CO.,LTD.
SPF grade Kunming mice: Division of Laboratory Animal of China Medical University
Adenine: Amresco, batch No. 20090514

### Example 1 Preparation of capsules

200 g of levocamitine, 150 g of calcium dobesilate, 98 g of microcrystalline cellulose, and 100 g of starch were comminuted separately through 80 mesh screens, and mixed homogeneously. The soft material was prepared with 10% polyvinylpyrrolidone solution, passed through a 24 mesh screen, dried at 50°C, and then granulated with 24 mesh screen. Magnesium stearate, talc, sodium carboxymethyl starch were added and mixed homogeneously, and then encapsulated. 2000 capsules were obtained in total.

### Example 2 Preparation of tablets

100 g of levocarnitine, 67 g of calcium dobesilate, 100 g of microcrystalline cellulose, 50 g of starch and 50 g of calcium sulfate were comminuted separately through 80 mesh screens, and mixed homogeneously. The soft material was prepared with pure ethanol, passed through a 24 mesh screen, dried at 50°C, and then granulated with 24 mesh screen. Magnesium stearate, silica gel powder, and low substituted hydroxypropyl cellulose were added and mixed homogeneously, and then pressed. 1000 tablets were obtained.

### Example 3 Preparation of oral solutions

50 g of levocarnitine and 20 g of sodium pyrosulfite were dissolved in suitable amount of purified water. 50g of sodium benzoate and 500 g of calcium dobesilate were successively added thereto. After the solution turned clear, its pH was adjusted with 0.1M hydrochloric acid and 0.1M sodium hydroxide. Purified water was then added to 10000ml and the solution was stirred homogeneously and filled into brown bottles (10ml / bottle), which were then charged with N₂. 1000 bottles of oral solutions were obtained.

### Example 4 Preparation of injections

250 g of mannitol was dissolved in suitable amount of water for injection. 100 g of levocarnitine and 100 g of calcium dobesilate were added thereto successively and stirred to dissolve. Its pH was adjusted to about 5.0 with 0.1M hydrochloric acid and 0.1M sodium hydroxide. Thereto water for injection was added to 2500 ml. The solution was stirred homogeneously, filled into Cillin-glass bottles (2.5 ml / bottle) and then freeze-dried with lyophilizer. 1000 injections were obtained.

### Example 5 Pharmacodynamic test-chronic renal failure model

Based on intergroup consistency principle, SPF grade Kunming mice (with a body weight of about 20 to 22g, half male and half female) were randomly divided into 5 groups (n=10) with one of them as the control group. Except for control group, 300 mg / kg (20 ml / kg●d) of adenine was administrated given to mice once per day for successive 3 days by gavage, thereby to obtain adenine-induced mice renal failure model as described in the references *(see* for example Pingdong Zheng et. al, "The establishment of chronic renal failure animal model with adenine, Chinese Journal of Nephrology, 1989, 5(4) : 342-344; Deyu Guo et.al., "The establishment of mice model with chronic renal insufficiency", CHINESE JOURNAL OF LABORATORY ANIMAL SCIENCE, 2001, 11(3) : 142-145; and Shuling Sun et. al, "The traditional Chinese medicinal intervention and pathological observations in mice chronic renal failure model", ZHEJIANG JOURNAL OF INTEGRATED TRADITIONAL CHINESE AND WESTERN MEDICINE, 2002, 12(4) : 221-222)..

Thereafter, except for the control group, based on intergroup consistency principle, the remaining 4 groups were redivided randomly into 4 groups, renal failure group, levocarnitine (350 mg / kg) group, calcium dobesilate (350 mg / kg) group, as well as levocarnitine and calcium dobesilate group (350 mg / kg) (wherein the ratio of levocarnitine and calcium dobesilate is 1:1). Except for the renal failure group, mice of each group were administrated at the given dosage once per day for successive 4 weeks. Then the data for the mice of each group such as body weight, blood urea nitrogen (BUN), serum creatinine (Scr), renal index and water content of renal tissue were determined. The results were given in the following table.

| Group | Control group | Renal failure group | Levocarnitine | Calcium dobesilate | levocarnitine+calcium dobesilate |
|---|---|---|---|---|---|
| initial body weight(g) | 24.2±3.0 | 24.8±5.1 | 24.9±5.0 | 24.6±4.9 | 24.8±5.4 |
| body weight after 4 weeks(g) | 40.3±3.1 | 32.9±3.9 | 32.3±5.3 | 31.6±4.6 | 35.1±6.1 |
| serum creatinine (Scr) (µmol/l) | 5.4±3.6 | 30.8±16.9 | 21.6±11.8 | 10.0±9.2 | 10.0±8.3 |
| blood urea nitrogen (BUN) (mmol/l) | 5.9±3.2 | 16.8±10.1 | 9.7±5.8 | 11.8±7.0 | 8.99±4.7 |
| renal index | 6.9±0.8 | 8.0±1.0 | 7.0±1.3 | 7.2±0.7 | 7.1±0.7 |
| Water content of renal tissue (%) | 68.2±4.1 | 78.6±4.6 | 76.0±2.0 | 75.9±1.8 | 74.8±1.6 |

From the results of serum creatinine in the above table, it can be seen that the Scr value of the renal failure group was significantly higher than that of the control group as compared with the control group, indicating the successful establishment of this model. As compared with the renal failure group, the values of Scr and BUN of both the levocarnitine group and the calcium dobesilate group decreased and demonstrated that the levocarnitine group and calcium dobesilate group both had therapeutic effects for renal failure. The comparison of the levocarnitine and calcium dobesilate group with the levocarnitine group and the calcium dobesilate group respectively demonstrated that the levocarnitine and calcium dobesilate group had better therapeutic effects (P<0.01), which means that the combination of levocarnitine and calcium dobesilate is superior to the use of levocarnitine or calcium dobesilate alone, and thus has synergistic effects for modulating the increased level of serum creatinine and/or blood urea nitrogen, and further for the treatment of renal failure.

### Example 6 Test on the ratio of active components

According to the procedure of example 4, the mice were divided into control group, renal failure group and 7 test groups with different ratios of levocarnitine and calcium dobesilate (n=10) (with the mass ratio of levocarnitine to calcium dobesilate being 1:10, 1:6, 1:3, 1:1, 3:1, 6:1 and 10:1, respectively) to perform the test (with the total amount used of levocarnitine and calcium dobesilate being 350mg/kg).

| Group No. | Ratio of levocarnitine to calcium dobesilate | BUN(mmol/l) | Scr(µmol/l) |
|---|---|---|---|
| Control group | - | 6.1±3.7 | 8.2±8.4 |
| renal failure group | - | 15.2±11.4 | 34.3±30.2 |
| Group 1 | 1:10 | 13.1±11.0 | 8.8±6.5 |
| Group 2 | 1:6 | 12.7±9.9 | 9.1±7.6 |
| Group 3 | 1:3 | 10.0±8.6 | 10.3±9.5 |
| Group 4 | 1:1 | 9.1±10.6 | 10.8±8.2 |
| Group 5 | 3:1 | 8.3±5.7 | 14.3±22.2 |
| Group 6 | 6:1 | 7.7±5.9 | 22.2±14.5 |
| Group 7 | 10:1 | 7.5±6.5 | 25.7±16.3 |

From the above-mentioned results of BUN and Scr, it can be seen that by the comparison of the renal failure group with the control group, the values of BUN and Scr for the renal failure group were notably higher than those of the control group, indicating the successful establishment of the model. By the comparison of groups 1∼7 with the renal failure group, the values of Scr and BUN all decreased, and demonstrated that the ratios of levocarnitine and calcium dobesilate within the range from 1:10 to 10:1 were all effective, preference is given to the ratio of about 1:1. Based on the above experimental results, the ratio of levocarnitine and calcium dobesilate was preferably between about 1:2 and 2:1 by statistical analysis.

### Example 7 Toxicity test

According to the methods described in literatures ("Guiding principles for acute toxicity test of a chemical drug", No. [H]GPT1-1, CENTER FOR DRUG EVALUATION, SFDA; Bojun Yuan et. al., "The Preclinical Safety Evaluation and Practice of New Drug", Military Medical Science Press, 1997: 23-36; Bojun Yuan et. al., "Methods and Techniques for Drug ToxicologicalTest", Chemical Industry Press, 2008: 200-210), the preparation of levocarnitine and calcium dobesilate obtained from example 2 was tested for mice acute toxicity. The results showed the LD₅₀ value of the preparation tested was 17.0g/kg. As compared with each of the LD₅₀ value of levocarnitine and calcium dobesilate (LD₅₀ of levocarnitine was 19.2g/kg, LD₅₀ of calcium dobesilate was 10.6g/kg), the toxicity of the combination of levocarnitine and calcium dobesilate showed no increase, and no additional toxicity target organic was observed, which demonstrates that the pharmaceutical composition according to the present invention has a good safety.

While the invention has been further illustrated with reference to the above specific embodiments, it should be understood that various deletions, substitutions and modifications can be made by a person skilled in the art without departing from the spirit and scope of the invention defined by the appended claims. As such, all such deletions, substitutions and modifications are believed to be within the spirit and scope of the invention.

## Claims

1. A pharmaceutical composition comprising levocarnitine and dobesilate.

2. The pharmaceutical composition according to claim 1, which consists of levocarnitine and dobesilate together with a pharmaceutically acceptable excipient.

3. The pharmaceutical composition according to claim 1 or 2, wherein the levocarnitine is selected from acid addition salts of levocarnitine, such as hydrochloride, hydrobromide, hydriodide, hydrofluoride, sulfate, citrate, maleate, acetate, lactate, nicotinate, succinate, oxalate, phosphate, malonate, salicylate, phenylacetate, stearate, methanesulfonate, picrate, tartarate and the like; base addition salts of levocarnitine, such as pyridine salts, ammonium salts, piperazine salts, diethylamine salts, niacinamide salts, formate, alkali metal salts, alkaline earth metal salts, cinnamate, methylamine salts, triethylamine salts, dimethylamine salts, tris(methylol)aminomethane salts and the like, and levocarnitine inner salts.

4. The pharmaceutical composition according to claim 1 or 2, wherein the dobesilate is selected from the group consisting of the pyridine, ammonium, piperazine, diethylamine, niacinamide, formic acid, alkali metal, alkaline earth metal, cinnamic acid, methylamine, triethylamine, dimethylamine, tris(methylol)aminomethane salt of dobesilate and the like, preferably selected from sodium salts, potassium salts, calcium salts, magnesium salts, zinc salts and lithium salts of dobesilate.

5. A use of levocarnitine and dobesilate in the preparation of a medicament for regulating the level of serum creatinine and/or blood urea nitrogen.

6. A use of levocarnitine and dobesilate in the preparation of a medicament for treating and/or preventing diseases and/or disorders influencing renal function, for example, including but not limited to various primary and secondary nephritis, renal lesions, renal insufficiency, nephritic syndrome, and even renal failure and uremia, as well as complications induced by the diseases and/or disorders influencing renal function, for example cardiovascular diseases, diabetes and the like.

7. The use according to claim 5 or 6, wherein the levocarnitine is selected from acid addition salts of levocarnitine, such as hydrochloride, hydrobromide, hydriodide, hydrofluoride, sulfate, citrate, maleate, acetate, lactate, nicotinate, succinate, oxalate, phosphate, malonate, salicylate, phenylacetate, stearate, methanesulfonate, picrate, tartarate and the like; base addition salts of levocarnitine, such as pyridine salts, ammonium salts, piperazine salts, diethylamine salts, niacinamide salts, formate, alkali metal salts, alkaline earth metal salts, cinnamate, methylamine salts , triethylamine salts, dimethylamine salts, tris(methylol)aminomethane salts and the like, and levocarnitine inner salts.

8. The use according to claim 5 or 6, wherein the dobesilate is selected from the group consisting of the pyridine, ammonium, piperazine, diethylamine, niacinamide, formic acid, alkali metal, alkaline earth metal, cinnamic acid, methylamine, triethylamine, dimethylamine, tris(methylol)aminomethane salt of dobesilate and the like, preferably selected from sodium salts, potassium salts, calcium salts, magnesium salts, zinc salts and lithium salts of dobesilate.

9. The use according to claim 5 or 6, wherein the medicament is formulated into an injection or a dosage form for oral administration, such as tablets, capsules, or oral solutions.

10. A method for regulating the level of serum creatinine (Scr) and/or blood urea nitrogen (BUN) comprising administering the pharmaceutical composition according to any of claims 1-4 to a patient in need thereof.

11. A method for treating and/or preventing diseases influencing renal function comprising administering the pharmaceutical composition according to any of claims 1-4 to a patient in need thereof.

12. The method according to claim 11, wherein the diseases influencing renal function is selected from primary and secondary nephritis, renal lesions, renal insufficiency, nephritic syndrome, renal failure, uremia, and complications induced by the diseases influencing renal function, for example cardiovascular diseases, diabetes and the like.

13. The method according to claim 11, wherein the diseases influencing renal function is chronic renal failure.
